# DEMANDE DE BREVET EUROPEEN

(11) **EP 1 666 094 A1**
(43) Date de publication de la demande: **07.06.2006**
(21) Numéro de dépôt: 05292558.3
(22) Date de dépôt: 02.12.2005
(51) Int. Cl.: A61Q 5/06, A61Q 5/08, A61K 8/46, A61K 8/22

(54) **Composition pour la décoloration et la coloration simultanée des fibres kératiniques comprenant du 7-(6'-méthylphénylazo)-1-acétamido-3,6-disulfo-8-hydroxy-naphtalène**

(30) Priorité: 03.12.2004 FR 0452862
(71) Demandeur: L'OREAL, 75008 Paris (FR)
(72) Inventeur: Kravtchenko, Sylvain, 92600 Asnieres (FR); Bonnardel Valérie,, 92700 Colombes (FR)
(74) Mandataire: Prevel, Estelle Nicole

(57) **Abrégé**

La présente invention a pour objet une composition pour la décoloration et la coloration simultanée des fibres kératiniques comprenant au moins un colorant choisi parmi le 7-(6'-méthylphénylazo)-1-acétamido-3,6-disulfo-8-hydroxy-naphtalène et ses sels d'addition, au moins un sel peroxygéné et au moins un agent alcalin, le procédé de décoloration et de coloration des fibres kératiniques mettant en oeuvre cette composition, ainsi que l'utilisation de cette composition pour la décoloration et la coloration simultanée des fibres kératiniques.

La composition conforme à la présente invention est particulièrement bien adaptée à des cheveux foncés. Elle présente une stabilité améliorée dans le temps et permet d'obtenir une coloration chromatique et tenace.

## Description

La présente invention a pour objet une composition pour la décoloration et la coloration simultanée des fibres kératiniques, et en particulier des fibres kératiniques humaines telles que les cheveux, comprenant au moins un colorant choisi parmi le 7-(6'-méthylphénylazo)-1-acétamido-3,6-disulfo-8-hydroxy-naphtalène et ses sels d'addition, au moins un sel peroxygéné et au moins un agent alcalin.

Lorsqu'une personne souhaite changer radicalement de couleur de cheveux, notamment lorsqu'elle souhaite obtenir une couleur plus claire que sa couleur d'origine, il est souvent nécessaire de procéder à une décoloration, et éventuellement à une coloration des cheveux. Pour ce faire, il existe plusieurs méthodes.

La première méthode consiste à utiliser des produits éclaircissants à base d'ammoniaque et de peroxyde d'hydrogène. Ces produits peuvent éventuellement contenir des colorants ce qui permet d'éclaircir et de colorer simultanément les cheveux. Toutefois, les performances éclaircissantes de ces produits restent limitées, plus particulièrement pour des applications sur des cheveux à fonds foncés naturels et / ou colorés.

La deuxième méthode consiste à appliquer sur les cheveux une composition éclaircissante à base de sels peroxygénés tels que le persulfate et d'agents alcalins dans laquelle a été ajouté du peroxyde d'hydrogène au moment de l'emploi, afin d'obtenir un éclaircissement plus important. Ce type de produit est très satisfaisant et plus adapté à des fonds foncés, mais il ne conduit qu'à une gamme très restreinte de reflets. Il est alors nécessaire de corriger la nuance obtenue en appliquant dans un deuxième temps un produit de coloration sur les cheveux. Ce procédé en deux étapes présente l'inconvénient d'être relativement long.

Pour pallier cet inconvénient, il est connu d'ajouter à ces produits éclaircissants des colorants. Cette méthode permet de colorer et de décolorer simultanément la fibre capillaire. Le niveau d'éclaircissement étant important, elle est particulièrement bien adaptée à des fonds foncés naturels et / ou colorés. Cependant, il existe un nombre très réduit de colorants stables dans ces conditions très oxydantes ce qui limite la variété des reflets pouvant être obtenus. Par ailleurs, cette instabilité se traduit par une modification plus ou moins rapide du reflet durant l'application ce qui conduit à des résultats peu reproductibles.

De plus, la ténacité de ces colorants face aux agents extérieurs, en particulier la lumière et les shampooings, n'est pas satisfaisante.

Il a été proposé, dans le brevet US 5 688 291, la demande de brevet WO 02/074270 et le modèle d'utilité DE 203 03 559, des colorants directs de type anthraquinone, azo, triarylméthane, thiazine, quinone et nitro, qui sont stables dans ces milieux fortement oxydants. Ces colorants ne sont cependant pas satisfaisants en terme de chromaticité, de ténacité et de stabilité pendant le temps de pose.

Le but de la présente invention est de fournir de nouvelles compositions pour la décoloration et la coloration simultanée des fibres kératiniques, et en particulier des fibres kératiniques humaines telles que les cheveux, particulièrement bien adaptées à des fonds foncés, qui présentent une bonne stabilité dans le temps et permettent d'obtenir des colorations chromatiques et tenaces.

Ce but est atteint avec la présente invention qui a pour objet une composition pour la décoloration et la coloration simultanée des fibres kératiniques comprenant :
- au moins un colorant choisi parmi le 7-(6'-méthylphénylazo)-1-acétamido-3,6-disulfo-8-hydroxy-naphtalène et ses sels d'addition ;
- au moins un sel peroxygéné ; et
- au moins un agent alcalin.

La composition conforme à la présente invention est particulièrement bien adaptée pour la décoloration et la coloration simultanée des cheveux foncés. Elle présente une stabilité améliorée dans le temps et permet d'obtenir une coloration chromatique. De plus, avec des concentrations adaptées en colorants selon l'invention, on peut obtenir des reflets pastels.

Cette coloration est résistante aux diverses agressions que peuvent subir les cheveux telles que les shampoings, les frottements, la lumière, les intempéries, la sueur et les déformations permanentes. Elle est aussi puissante, esthétique, et de plus peu sélective, c'est-à-dire qu'elle permet d'obtenir de faibles écarts entre différentes parties différemment sensibilisées d'un cheveu ou d'une chevelure.

La présente invention a également pour objet un procédé de décoloration et de coloration simultanée des fibres kératiniques mettant en oeuvre la composition conforme à l'invention, ainsi que des dispositifs à plusieurs compartiments pour la mise en oeuvre de ce procédé.

Un autre objet de la présente invention est l'utilisation de la composition conforme à l'invention pour la décoloration et la coloration simultanée des fibres kératiniques.

Le 7-(6'-méthylphénylazo)-1-acétamido-3,6-disulfo-8-hydroxy-naphtalène et ses sels d'addition sont des colorants directs azoïques.

D'une manière générale, les sels d'addition du 7-(6'-méthylphénylazo) 1-acétamido 3,6-disulfo 8-hydroxy-naphtalène utilisables dans le cadre de l'invention sont notamment choisis parmi les sels d'addition avec une base organique ou minérale, et en particulier les sels des métaux alcalins ou alcalino-terreux et les sels d'amines organiques telles que les alcanolamines.

De préférence, le ou les colorants utiles dans le cadre de l'invention sont choisis parmi les sels de sodium du 7-(6'-méthylphénylazo) 1-acétamido 3,6-disulfo 8-hydroxy-naphtalène et leurs mélanges. Encore plus préférentiellement, le ou les colorants utiles dans le cadre de l'invention sont choisis parmi l'Acid Red 35, encore appelé Supramine Red 3BA, dont la structure est la suivante :

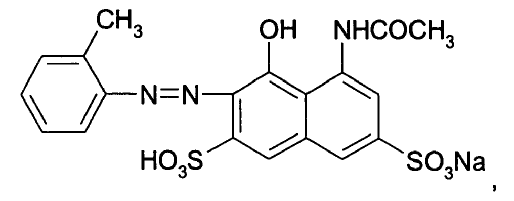

l'Acid Red 55 dont la structure est la suivante : 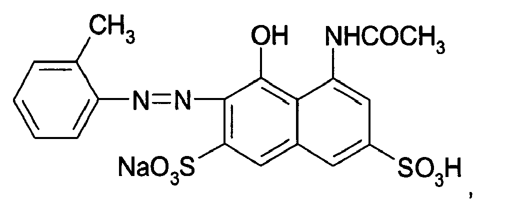 et leurs sels d'addition.

La concentration en 7-(6'-méthylphénylazo)-1-acétamido-3,6-disulfo-8-hydroxy-naphtalène et / ou ses sels d'addition dans la composition conforme à l'invention est généralement comprise entre 0,0001 et 10 % en poids, de préférence entre 0,001 et 8 %, et encore plus préféntiellement entre 0,01 et 5 % en poids du poids total de la composition.

Le ou les sels peroxygénés utiles à l'invention sont par exemple choisis parmi les persulfates, les perborates, les percarbonates, les peroxydes de métaux alcalins ou alcalino-terreux, et leurs mélanges. De préférence, on utilisera les persulfates et leurs mélanges, et plus préférentiellement les persulfates de sodium, de potassium et d'ammonium, et leurs mélanges.

La concentration en sels peroxygénés dans la composition conforme à l'invention est généralement comprise entre 10 et 70 % en poids, et de préférence entre 20 et 60 % en poids du poids total de la composition.

Le ou les agents alcalins utiles dans la composition de la présente invention sont par exemple choisis parmi l'urée, les sels d'ammonium comme le chlorure d'ammonium, le sulfate d'ammonium, le phosphate d'ammonium ou le nitrate d'ammonium, les silicates, les phosphates ou les carbonates de métaux alcalins ou alcalino-terreux, tels que le lithium, le sodium, le potassium, le magnésium, le calcium, le baryum, et leurs mélanges. De préférence, le ou les agents alcalins sont choisis parmi le chlorure d'ammonium, les silicates, les carbonates, et leurs mélanges.

La concentration en agents alcalins dans la composition conforme à l'invention est généralement comprise entre 0,01 et 40 % en poids, et de préférence entre 0,1 et 30 % en poids du poids total de la composition.

La composition conforme à l'invention peut se présenter sous forme de poudre ou de pâte. Avantageusement, la composition de l'invention se présente sous forme de pâte.

Dans le cas où la composition conforme à l'invention se présente sous forme de pâte, elle comprend de plus au moins une phase liquide inerte organique.

Par phase liquide, on entend au sens de la présente invention toute phase capable d'écoulement à température ambiante, généralement entre 15 °C et 40 °C, et à pression atmosphérique, sous l'action de son propre poids.

A titre d'exemple de phase liquide inerte, on peut citer les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales, ou leurs mélanges.

Les composés de formule C₁₀ₙ H_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo® 366 NF Polydecene par la société Amoco Chemical, ceux vendus sous la dénomination Nexbase® 2002 FG, 2004 FG, 2006 FG et 2008 FG par la société Fortum.

En ce qui concerne les esters d'alcools gras ou d'acides gras, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle et le stéarate d'octyl dodécyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de di-isopropyle,
- les esters de monoalcools linéaires ou ramifiés en C₃-C₈, avec des acides gras bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme phase liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme phase liquide inerte organique, comme par exemple l'huile de paraffine.

Les huiles végétales peuvent aussi convenir, et notamment l'huile d'avocat, l'huile d'olive ou la cire liquide de jojoba.

De préférence, la phase liquide inerte organique est choisie dans le groupe formé par les polydécènes de formule C₁₀ₙH_{[(20n)+2]} dans laquelle n varie de 3 à 9 et de préférence de 3 à 7, les esters d'alcools gras ou d'acides gras, et leurs mélanges.

Selon un mode de réalisation particulier de l'invention, la teneur en phase liquide inerte organique varie de 5 à 60 % en poids, de préférence de 10 à 50 % en poids par rapport au poids de la pâte anhydre, et encore plus préférentiellement de 15 à 45 %.

Selon un mode de réalisation particulier de l'invention, la composition conforme à l'invention est anhydre.

Dans le cadre de la présente invention, une composition est anhydre lorsqu'elle présente une teneur en eau inférieure à 1 % en poids, et de préférence inférieure à 0,5 % en poids par rapport au poids total de la composition.

Selon un autre mode de réalisation particulier de l'invention, la composition conforme à l'invention comprend de plus du peroxyde d'hydrogène.

Le pH de la composition contenant du peroxyde d'hydrogène conforme à l'invention est généralement compris entre les valeurs 3 et 11. Il est de préférence compris entre 7 et 11.

La composition conforme à la présente invention peut également comprendre divers additifs classiquement utilisés en cosmétique.

La composition conforme à la présente invention peut ainsi comprendre des agents épaississants minéraux ou organiques, et en particulier des polymères épaississants associatifs ou non, anioniques, cationiques, non ioniques ou amphotères, des charges telles que des argiles, des liants tels que la vinylpyrrolidone, des lubrifiants comme les stéarates de polyol ou les stéarates de métaux alcalins ou alcalino-terreux, des silices hydrophiles ou hydrophobes, des pigments, des colorants autres que ceux de la présente invention, des agents matifiants comme les oxydes de titane ou encore des agents tensioactifs anioniques, non ioniques, cationiques, amphotères ou zwittérioniques, des agents antioxydants, des agents de pénétration, des agents séquestrants, des tampons, des agents dispersants, des agents filmogènes, des agents conservateurs, des agents opacifiants, des vitamines, des parfums, des polymères anioniques, cationiques, non ioniques, amphotères ou zwittérioniqes, des céramides, des agents de conditionnement tels que par exemple des silicones volatiles ou non volatiles, modifiées ou non modifiées.

Dans le cas où la composition conforme à l'invention comprend du peroxyde d'hydrogène, elle peut également comprendre des agents de contrôle du dégagement d'oxygène tels que le carbonate ou l'oxyde de magnésium.

Les additifs et les agents de contrôle du dégagement d'oxygène tels que définis précédemment peuvent être présents en quantité comprise pour chacun d'eux entre 0,01 et 40 % en poids, de préférence entre 0,1 et 30 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le procédé de décoloration et de coloration simultanée conforme à la présente invention consiste à appliquer sur les fibres kératiniques une composition contenant du peroxyde d'hydrogène conforme à l'invention telle que définie précédemment.

La présente invention a également pour objet un dispositif à plusieurs compartiments, caractérisé par le fait qu'il contient au moins deux compositions dont le mélange conduit à une composition contenant du peroxyde d'hydrogène conforme à l'invention telle que définie précédemment.

Selon un mode de réalisation particulier de l'invention, le dispositif conforme à invention comporte un premier compartiment qui contient une composition (A) comprenant, dans un milieu approprié pour la teinture, au moins un colorant tel que défini précédemment, un deuxième compartiment qui contient une composition (B) anhydre comprenant au moins un sel peroxygéné et au moins un agent alcalin tels que définis précédemment, et un troisième compartiment qui contient une composition (E) aqueuse de peroxyde d'hydrogène.

Selon un autre mode de réalisation particulier de l'invention, le dispositif conforme à invention comporte un premier compartiment qui contient une composition (C) anhydre comprenant au moins un colorant tel que défini précédemment, au moins un sel peroxygéné et au moins un agent alcalin tels que définis précédemment, et un deuxième compartiment qui contient une composition (E) aqueuse de peroxyde d'hydrogène.

Selon un autre mode de réalisation particulier de l'invention, le dispositif conforme à invention comporte un premier compartiment qui contient une composition (B) anhydre comprenant au moins un sel peroxygéné et au moins un agent alcalin tels que définis précédemment, et un deuxième compartiment qui contient une composition (D) comprenant, dans un milieu approprié pour la teinture, au moins un colorant tel que défini précédemment et du peroxyde d'hydrogène.

Le milieu approprié pour la teinture des compositions (A) et (D) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition tinctoriale, et plus préférentiellement encore entre 5 et 30 % en poids environ.

La composition (A), encore appelée "booster", peut être formulée à pH acide, neutre ou alcalin, le pH pouvant varier entre 3 et 12 environ et de préférence entre 4 et 11 environ.

La composition (D) présente de préférence un pH inférieur à 7, le pH acide garantissant la stabilité du peroxyde d'hydrogène dans cette composition.

Les compositions (A) et (D) peuvent se présenter sous des formes diverses, telles que sous forme de liquides, de crèmes, de gels, ou sous toute autre forme appropriée pour réaliser une teinture des fibres kératiniques.

Les compositions (B) et (C) anhydres peuvent se présenter sous forme de poudre ou de pâte. Dans ce cas-là, elles comprennent de plus une phase liquide inerte organique telle que définie précédemment.

La composition (E) aqueuse de peroxyde d'hydrogène présente de préférence un pH inférieur à 7, le pH acide garantissant la stabilité du peroxyde d'hydrogène dans cette composition.

Les compositions (A), (B), (C), (D) et (E) peuvent également renfermer divers additifs classiquement utilisés en cosmétique tels que ceux qui sont décrits précédemment.

Les compositions (E) et (D) peuvent de plus comprendre des agents de contrôle du dégagement d'oxygène tels que définis précédemment.

Le dispositif conforme à la présente invention peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de décolorer et de colorer simultanément les fibres kératiniques à partir d'un procédé conforme à l'invention tel que défini précédemment.

La présente invention a aussi pour objet l'utilisation pour la décoloration et la coloration simultanée des fibres kératiniques d'une composition conforme à l'invention telle que définie précédemment.

Les exemples qui suivent servent à illustrer l'invention sans toutefois présenter un caractère limitatif.

### EXEMPLE

L'Acid Red 35 est solubilisé dans une solution hydroalcoolique (80 / 20) à la concentration de 3,5 g %. On obtient ainsi le booster A.
Cette solution est ajoutée juste avant utilisation à un mélange comprenant de la poudre décolorante Platine Précision B comprenant 51,5 % d'un mélange de persulfates de sodium, de potassium et de magnésium en présence de 4,2 % d'un mélange de métasilicate de sodium et de chlorure d'ammonium et un oxydant E constitué par une composition aqueuse de peroxyde d'hydrogène à 40 volumes. Les proportions du mélange poudre décolorante B / oxydant E / booster A sont respectivement 1 / 2 / 0,5.
Une partie de ce mélange est appliqué immédiatement sur une mèche de cheveux naturels 90 % blancs de 1 g ainsi que sur une mèche naturelle chatain de 2,7 g.
Le restant de ce mélange est appliqué 20 minutes après sur une mèche de cheveux naturels 90 % blancs de 1 g ainsi que sur une mèche de cheveux naturels chatains de 2,7 g.
Dans tous les cas, les conditions sont identiques. On utilise 10 g de composition pour 1 g de cheveux.
Après un temps de pose de 30 minutes, les mèches sont rincées puis shampooinées, rincées à nouveau et séchées.
Les résultats sont décrits dans le tableau ci-dessous.

| **REFLETS OBTENUS SUITE A L'APPLICATION DES COMPOSITIONS DE l'INVENTION** | | |
|---|---|---|
| | Cheveux naturels 90 % blancs | Cheveux naturels chatains |
| **Application immédiate du mélange** | Rose pâle | Cuivré nacré chromatique |
| **Application différée du mélange** | Rose pâle | Cuivré nacré chromatique |

Les cheveux naturels 90 % blancs sont utilisés ici pour exacerber l'éventuelle modification de reflet.
On constate que l'on obtient le même reflet dans le cas d'une application du mélange immédiate et dans le cas d'une application différée.
Ces résultats montrent que les compositions conformes à l'invention sont stables dans le temps.

## Revendications

1. Composition pour la décoloration et la coloration simultanée des fibres kératiniques comprenant :
- au moins un colorant choisi parmi le 7-(6'-méthylphénylazo)-1-acétamido-3,6-disulfo-8-hydroxy-naphtalène et ses sels d'addition ;
- au moins un sel peroxygéné ; et
- au moins un agent alcalin.

2. Composition selon la revendication 1, dans laquelle le ou les colorants sont choisis parmi les sels de sodium du 7-(6'-méthylphénylazo)-1-acétamido-3,6-disulfo-8-hydroxy-naphtalène et leurs mélanges.

3. Composition selon la revendication 2, dans laquelle le ou les colorants sont choisis parmi l'Acid Red 35, l'Acid Red 55, et leurs mélanges.

4. Composition selon l'une quelconque des revendications précédentes, dans laquelle la concentration en 7-(6'-méthylphénylazo)-1-acétamido-3,6-disulfo-8-hydroxy-naphtalène et / ou ses sels d'addition est comprise entre 0,0001 et 10 % en poids du poids total de la composition.

5. Composition selon l'une quelconque des revendications précédentes, dans laquelle le ou les sels peroxygénés sont choisis parmi les persulfates, les perborates, les percarbonates, les peroxydes de métaux alcalins ou alcalino-terreux, et leurs mélanges.

6. Composition selon la revendication 5, dans laquelle le ou les sels peroxygénés sont choisis parmi les persulfates et leurs mélanges.

7. Composition selon la revendication 6, dans laquelle le ou les sels peroxygénés sont choisis parmi le persulfate de sodium, le persulfate de potassium, le persulfate d'ammonium, et leurs mélanges.

8. Composition selon l'une quelconque des revendications précédentes, dans laquelle la concentration en sels peroxygénés est comprise entre 10 et 70 % en poids du poids total de la composition.

9. Composition selon l'une quelconque des revendications précédentes, dans laquelle le ou les agents alcalins sont choisis parmi l'urée, le chlorure d'ammonium, le sulfate d'ammonium, le phosphate d'ammonium, le nitrate d'ammonium, les silicates, les phosphates ou les carbonates de métaux alcalins ou alcalino-terreux, et leurs mélanges.

10. Composition selon l'une quelconque des revendications précédentes, dans laquelle la concentration en agents alcalins est comprise entre 0,01 et 40 % en poids du poids total de la composition.

11. Composition selon l'une quelconque des revendications précédentes, comprenant de plus au moins une phase liquide inerte organique.

12. Composition selon la revendication 11, dans laquelle la ou les phases liquides inertes organiques sont choisies parmi par les polydécènes de formule C₁₀ₙH_{[(20n)+2]} dans laquelle n varie de 3 à 9, les esters d'alcools gras ou d'acides gras, les esters ou di-esters de sucres d'acides gras en C₁₂-C₂₄, les éthers cycliques ou les esters cycliques, les huiles de silicone, les huiles minérales, les huiles végétales, et leurs mélanges.

13. Composition selon la revendication 12, dans laquelle la ou les phases liquides inertes organiques sont choisies parmi les polydécènes de formule C₁₀ₙH_{[(20n)+2]} dans laquelle n varie de 3 à 9, les esters d'alcools gras ou d'acides gras, et leurs mélanges.

14. Composition selon l'une quelconque des revendications 11 à 13, dans laquelle la concentration en phases liquides inertes organiques est comprise entre 5 et 60 % en poids du poids total de la composition.

15. Composition selon l'une quelconque des revendications précédentes, **caractérisée en ce qu'**elle est anhydre.

16. Composition selon l'une quelconque des revendications 1 à 14, comprenant de plus du peroxyde d'hydrogène.

17. Procédé de décoloration et de coloration simultanée des fibres kératiniques, **caractérisé par le fait que** l'on applique sur lesdites fibres kératiniques une composition telle que définie à la revendication 16.

18. Dispositif à plusieurs compartiments, **caractérisé par le fait qu'**il contient au moins deux compositions dont le mélange conduit à une composition telle que définie à la revendication 16.

19. Dispositif selon la revendication 18, dans lequel un premier compartiment contient une composition (A) comprenant, dans un milieu approprié pour la teinture, au moins un colorant tel que défini à l'une quelconque des revendications 1 à 3, un deuxième compartiment contient une composition (B) anhydre comprenant au moins un sel peroxygéné tel que défini à l'une quelconque des revendications 1 et 5 à 7 et au moins un agent alcalin tel que défini à la revendication 1 ou 9, et un troisième compartiment contient une composition (E) aqueuse de peroxyde d'hydrogène.

20. Dispositif selon la revendication 18, dans lequel un premier compartiment contient une composition (C) anhydre comprenant au moins un colorant tel que défini à l'une quelconque des revendications 1 à 3, au moins un sel peroxygéné tel que défini à l'une quelconque des revendications 1 et 5 à 7 et au moins un agent alcalin tel que défini à la revendication 1 ou 9, et un deuxième compartiment contient une composition aqueuse (E) de peroxyde d'hydrogène.

21. Dispositif selon la revendication 18, dans lequel un premier compartiment contient une composition (B) anhydre comprenant au moins un sel peroxygéné tel que défini à l'une quelconque des revendications 1 et 5 à 7 et au moins un agent alcalin tel que défini à la revendication 1 ou 9, et un deuxième compartiment contient une composition (D) comprenant, dans un milieu approprié pour la teinture, au moins un colorant tel que défini à l'une quelconque des revendications 1 à 3 et du peroxyde d'hydrogène.

22. Utilisation pour la décoloration et la coloration simultanée d'une composition telle que définie à l'une quelconque des revendications 1 à 16.
